**Europäisches Patentamt**

(19) **European Patent Office**

**Office européen des brevets**

(11) Publication number: **0 341 396 B1**

## (12) EUROPEAN PATENT SPECIFICATION

(45) Date of publication of patent specification :
03.06.92 Bulletin 92/23

(51) Int. Cl.⁵: **B65B 55/02, A61L 2/20**

(21) Application number : **89104508.0**

(22) Date of filing : **14.03.89**

(54) **Sterilization method for a packing machine that uses liquid disinfectant.**

(30) Priority : **27.04.88 JP 107384/88**

(43) Date of publication of application :
**15.11.89 Bulletin 89/46**

(45) Publication of the grant of the patent :
**03.06.92 Bulletin 92/23**

(84) Designated Contracting States :
**AT BE CH DE ES FR GB IT LI NL SE**

(56) References cited :
**FR-A- 2 366 842**

(73) Proprietor : **TETRA DEV-CO**
**Via Delfini 1**
**I-411 00 Modena (IT)**

(72) Inventor : **Bordini, Giorgio**
**Via Luppi 8 S. Prospero**
**I-41100 Modena (IT)**

(74) Representative : **Glawe, Delfs, Moll & Partner**
**Patentanwälte**
**Postfach 26 01 62 Liebherrstrasse 20**
**W-8000 München 26 (DE)**

## Description

This invention relates to a sterilization method for sterilizing surfaces of components of a machine for filling, dispensing and/or for packing of sterile liquids by using a liquid disinfectant.

In the manufacture of single-life package containers sterile-packed with liquid products such as milk or juice the web material that packs such contents is bent as it passes through a packing machine after sterilization, and the lengthwise edges of said material slightly overlap mutually as a seal thus forming a tube, whereby said contents are supplied continuously from a filler pipe extending into the upper open end of said tube. As said tube travels lower, the tube is sealed as its prescribed position is pushed flat, whereby the contents level is automatically controlled so that it is sufficiently above the position where the seal comes. Once sealed the tube separates horizontally at the sealed position, and the manufacturing process is completed after the prescribed reforming.

For a packing machine of the type described above, filling proceeds under sterilized conditions for sterile-packing manufacture. Thus, before executing filling operations at the packing machine, sterilization of components such as the filler pipe for the contents, the piping for feeding contents to the filler pipe, a sterile-air inlet pipe and piping connected thereto to feed sterile air so that unsterile air does not intrude from the outer side of the filler pipe onto the upper part of the contents in the web tube, and of other parts that require sterilization is performed. Conventional machines had these parts sterilized with high-temperature heated air, but recent machines sterilize with the vaporized steam of hydrogen peroxide at a lower temperature to avoid the ill effects of the heat toward valve packings along the piping and web. Sterilization with hydrogen peroxide involves the spraying of liquid hydrogen peroxide (aqueous solution of hydrogen peroxide) by opening a nozzle at a mixing chamber established along the piping that feeds heated air, and the forming of a steam, whereby a mixture of said steam and heated air is fed to filler piping, piping, and other parts requiring sterilization. For example, in order to disinfect filler pipes and piping requiring sterilization, the method described in Jap. Pat. Pub. 53-47548, identical to FR-A-2.366.842, (the embodiment here being disinfecting web) involves disinfectant spray-mixed and vaporized with a high-temperature air stream where the mixed-air dew point has a higher temperature than the surfaces to be disinfected, so that disinfectant cools and evenly condenses in layers over said surfaces to be disinfected by traveling to said surfaces, and whereby disinfectant is subsequently removed. In application, the surface temperatures of parts requiring sterilization are cooled initially from the high temperatures generated from preheating operations of the packing machine, etc., followed by sterilization with a mixture of hydrogen peroxide steam and heated air.

## PROBLEMS TO BE SOLVED BY THE INVENTION

In a conventional sterilization method using hydrogen peroxide to sterilize prescribed parts of a packing machine like the one described above, cooling of said parts (10 - 15 minutes) was required prior to sterilization so that the parts requiring sterilization had a lower temperature than the dew point of the mixture of disinfectant and air. Thus, a loss of time from cooling and increasing costs as a result therefrom are unavoidable.

The present invention takes notice of the above points, and provides a sterilization method that eliminates the cooling operation where the temperature of the surfaces to be sterilized is lowered. Hence, the mixture of liquid disinfectant steam and heated air clads and condenses on the surfaces to be sterilized without cooling the surfaces, and further, an efficient vaporization method of the liquid disinfectant is provided, eliminating time loss that occurred with the use of liquid disinfectant in packing machines, improving operation efficiency, and lowering cost.

## MEANS OF SOLVING THE PROBLEM

The above objectives are solved by a method according to claim 1 such a sterilization method uses piping that permits large-volume heat storage when heated air is passed through it, and after preheating said heat-storage piping by passing high-temperature heated air through it, a large volume of liquid disinfectant is spray-charged for a prescribed time into the prescribed high-temperature heated air passing through said heat-storage piping and is vaporized, whereby the mixture of vaporized disinfectant steam and heated air has its temperature decrease suppressed and said mixture of steam and heated air is established continually in the prescribed heat-storage piping so that without cooling said mixture contacts and passes through piping to be sterilized at a lower temperature than the dew point of said mixture, thus evenly cladding and condensing on inner surfaces of components requiring sterilization, and wherein, finally, heated air at a lower prescribed temperature than the high-temperature heated air noted above passes through and contacts the condensed disinfectant which is vaporized and removed.

The surface temperatures of components to be sterilized are at a temperature lower than the dew point of the mixture of disinfectant and heated air, in particular in the maximum range of 70°C to 90°C, and the dew point of said mixture needs to be 90°C or greater.

Preferably, spraying of the liquid disinfectant should work by positioning the nozzle opening next to the disinfectant supply pipe and by opening the throat of a venturi tube located in the prescribed heat-storage piping.

Also preferably, the thickness of the prescribed heat-storage piping should be a specific thickness thicker than the piping to be disinfected. In this case, establishing heat-exchanging materials such as radial fins in the thick heat-storage piping is satisfactory.

Spray-charging for 15 ~ 20 seconds is satisfactory.

Moreover, hydrogen peroxide is most appropriate as the liquid disinfectant used for this device, and ordinarily obtained aqueous hydrogen peroxide solution is used.

High-temperature heated air used for preheating the prescribed heat-storage piping is exhausted from the link between the heat-storage piping and piping to be sterilized so that it does not flow to the piping to be sterilized.

ACTION

With the sterilization method of the present invention arranged as described above, the heat-storage piping to be preheated stores a large volume of heat by passing high-temperature heated air. Thus, when a large amount of liquid disinfectant is spray-charged for a prescribed time into the flow of high-temperature heated air in said heat-storage piping, the liquid disinfectant vaporizes and becomes steam whereby the heat of its vaporization is absorbed so that the mixture of liquid-disinfectant steam and heated air attempts to significantly decrease in temperature; however, said heat of vaporization is restored by the heat stored in the heat-storage piping at vaporization via the interior walls of said piping, and temperature decrease is suppressed. For this reason, a dew-point decrease of said mixture, which fluctuates according to the air temperature and the ratio of disinfectant volume to air volume, is prevented, and said dew point rises compared to when heat of vaporization is not restored from heat-storage piping walls. When said mixture is continually established and contacts sterilizing objects such as piping to be sterilized as it passes, said objects are ordinarily at a lower temperature than the dew point of said mixture so the mixture cools on the surfaces of the parts requiring sterilization such as the inner surfaces of said piping whereby the disinfectant steam condenses and forms an even cladding on said surfaces. Later heated air of a prescribed temperature passes and contacts the condensed disinfectant which then evaporates and is removed, and said surfaces are sterilized.

If surface temperatures of piping interiors, etc., to be sterilized are in the maximum range of 70° to 90°C, this permits packing machines to ordinarily stand without cooling. If the dew point of the mixture in this case is 90°C or greater, disinfectant can be condensed smoothly on surfaces to be sterilized.

In addition, when spray-charging liquid disinfectant, vaporization proceeds rapidly by opening the throat of a venturi tube established in the heat-storage piping, since the flow speed of heated air increases and inner pressure decreases at the venturi tube throat.

Moreover, by increasing the piping wall thickness of the heat-storage piping thicker than the ordinary piping to be sterilized, the heat capacity becomes large and heat-storage capacity increases, thus easily preventing dew-point decrease of the mixture of disinfectant and heated air. The heat transfer toward said mixture is made efficient and fast by establishing heat-exchanging material such as radial fins in the heat-storage piping described above, and spray-charging for 15 to 20 seconds permits efficient operation.

In addition, hydrogen peroxide acts effectively as liquid disinfectant.

When preheating the heat-storage piping described above, the high-temperature heated air used is exhausted from the link between the heat-storage piping and piping to be sterilized, and completely prevents the temperature of the piping to be sterilized to rise.

EMBODIMENT

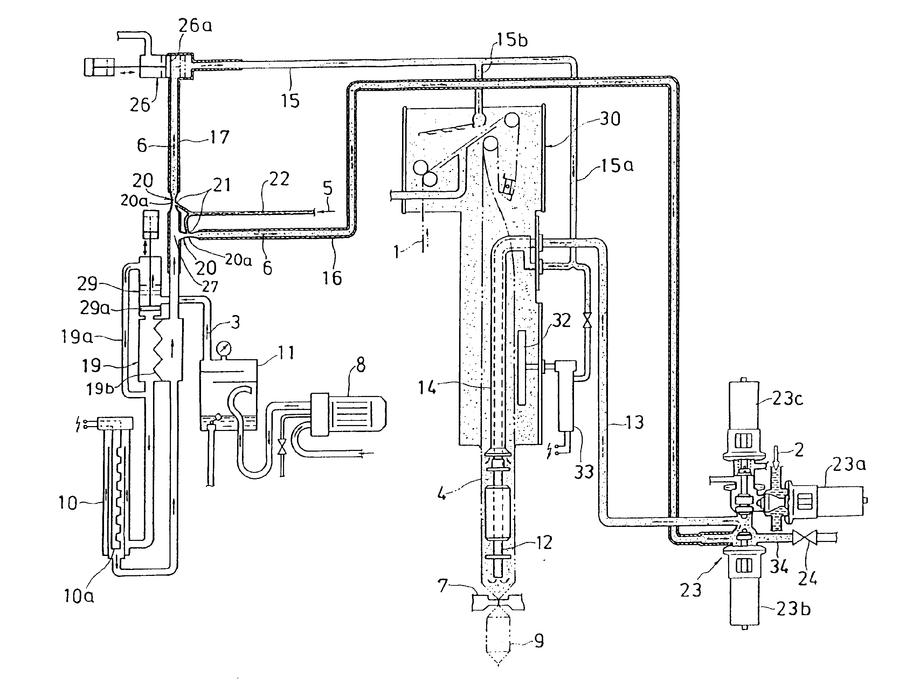

The present invention will now be described with reference to a preferred embodiment in conjunction with the accompanying drawing.

The drawing schematically shows a packing machine filling device utilizing the sterilization method of the present invention for sterilizing the prescribed components and shows the condition during liquid disinfectant spray-charging.

To simplify the understanding of the present invention, the basic principle of liquid disinfectant sterilization methods is described for background information.

Hence, when a specific amount of liquid disinfectant is charged into heated air, as long as the air temperature is sufficiently high and higher than the condensation temperature of the air mixture of the liquid disinfectant, the air will absorb and contain the disinfectant. When the air mixture that absorbs and contains the vaporized disinfectant steam decreases in temperature below the dew point which is the condensation temperature, the air cannot contain such a large amount of liquid, and a portion of said liquid condenses. The air temperature and ratio of liquid disinfectant volume to air volume for the air mixture containing the steam determine the dew point. Thus, contact with an object to be sterilized that is at a lower temperature than the dew point of the formed mixture causes the part of said mixture that contacts said surfaces to be cooled and the steam portion condenses and clads said surfaces with an even layer of liquid disinfectant. Later, heated air of a suitable temperature heats said surfaces and vaporizes the disinfectant to yield sterilization. The present invention efficiently sterilizes the prescribed parts of a packing machine to be sterilized based on the principle stated above.

For filling at the packing machine illustrated in the drawing, web 1 rolls off of an unillustrated roll, passes through a hydrogen peroxide bath and is sterilized, travels above prescribed guide rollers at the top of the packing machine, is dried, and travels vertically through the packing machine toward its lower parts with a regulated feed. During the lower movement through the packing machine, web 1 is formed into a tube after both lengthwise edges are sealed, and to the tube material 4 thus formed, prescribed sterilized contents such as milk are filled via filler pipe 12, which extends downward near the lower edge of material tube 4 where material tube 4 ends and beyond the upper open-mouth of material tube 4. The sealing jaws 7 located a certain distance below the lower end of filler pipe 12 on both sides of material tube 4 seal said tube 4 equally horizontally while said tube 4 is filled and traveling downward vertically, and said tube 4 is cut similarly horizontally and separated at said seal. The packaged object 9 thus formed is complete after prescribed reforming.

Prescribed parts of the packing machine are sterilized with liquid disinfectant prior to manufacture. Hydrogen peroxide is used as disinfectant. The packing machine and sterilization device according to the method of the present invention that operates in conjunction with the packing machine both connect to air compressor 8, which is equipped with a water separator 11 to remove condensed water from air. Since air contains small amounts of water, said water separator 11 prevents the dilution of disinfectant when mixed with the air, and removes water to the fullest extent for the air for preheating, drying and other operations. Dry air 3 thus dehydrated is heated and used for creating hydrogen peroxide steam by vaporizing hydrogen peroxide liquid disinfectant (normally obtained as 35% aqueous solution) which is charged in said air, for maintaining high temperatures and preventing the mixture temperature of hydrogen peroxide steam and heated air to decrease, and for preheating heat-storage piping 16, 17 described later. Dehydrated air 3 emitted from water separator 11 travels to heat-exchanger flow transfer valve 29 via prescribed piping. According to the operating conditions of the sterilization device and piston 29a in transfer valve 29, said transfer valve 29 switches flow paths for the spraying of hydrogen peroxide 5 and for sending heated air to heat-storage piping 16, 17 for preheating said heat-storage piping 16, 17 prior to said spraying, and for sending dry air at a lower temperature than the prescribed high-temperature air to piping to be sterilized connected via the valve device to vaporize the hydrogen peroxide 5 liquid disinfectant from piping to be sterilized such as piping inner surfaces. Hence, for sending high-temperature heated air to heat-storage piping 16, 17, piston 29a that moves up and down is down as shown in the figure, and dehydrated air 3 emitted from water separator 11 travels to high-temperature air heater 10 via the upper part of prescribed transfer valve 29 and side-path pipe 19a of heat exchanger 19. Heater 10a of said high-temperature heater heats said air 3 to approximately 360°C as it passes, whereby said air 3 passes heat exchanger 19 again and arrives at heat-storage piping 16, 17 via piping distribution point 27. For using heated air for drying, piston 29a noted above moves upward in the figure, and dry air 3 passes the interior of heat exchanger 19 and travels to high-temperature air heater 10, whereby high-temperature heated air passes by heat-exchanging plates 19b and is cooled by air 3 fed from water separator 11 to achieve an appropriate heated temperature, such that said air 3 feeds to piping 13, 15, which requires disinfectant to be vaporized and dried, via valves 23, 26 described later connected to heat-storage piping 16, 17. Venturi tubes connect to the sides of both pipes 16 and 17 that conduct air passed from heat exchanger 19 and that lead from distribution point 27. Pipe 16 (the lower pipe in the drawing) connects to filler pipe 12 of sterile production material 2 which is the liquid content to fill packaging containers, and to piping 13 connected to sterile production material valve device 23 that regulates supply to said piping 13. Pipe 17 (the upper pipe in the drawing) connects to heated-air flow-path transfer valve 26, which exhausts heated air according to operating conditions or which sends heated air to piping 15, whereby sterile air is sent to the upper part of tube-shaped web 4 during filling via sterile-air inlet pipe 14, connecting piping 15a and piping 15b, connected to sterile chamber 30. The sterile production material valve device 23 described above consists of production material valve 23a connected to the main product-line pipe and sterile-air valve 23b that opens and closes the path of sterilizing air mixture and cleaning liquid as described later, and steam barrier valve 23c that creates a sterilized steam barrier against said valve interior of main pipe described above, that switches the flow of sterile production material 2 from the product line, that also switches the flow of sterile air mixture 6 from heat-storage pipe 16. Hence during production, production material valve 23a is open, sterile-air valve 23b is closed, and production material 2 enters piping 13 through the valve device. During sterilization, however, the condition is as shown in the drawing.

At the throats 20a of respective venturi tubes 20, piping 22 that conducts pressurized hydrogen peroxide

5 from an unillustrated hydrogen peroxide tank via a pump has divided ends with open nozzles **21**. By movement of piston **26a**, heated air-path transfer valve **26** described above switches air flow from heat-storage pipe **17** to piping **15** on the side of sterile-air feed pipe **14** or to the external side. Thus, when conducting heated air to said piping **15**, piston **26a** which moves to the left and right moves to the left as shown in the drawing, and when exhausting heated air said piston **26a** moves to the right. Furthermore, to sterile-air valve **23b** of prescribed sterile production material valve device **23**, valve **24** is established next to pipe **34** which faces the open end of heat-storage pipe **16** and which connects to said pipe **16**. According to the operating conditions of the sterilization device, said valve **24** permits externally exhausting heated air used for preheating when preheating heat-storage pipe **16** with heated air prior to vaporizing hydrogen peroxide **5** in said piping **16**, while said valve **24** remains closed during all times except preheating to allow the transfer of heated air flowing from heat-storage piping **16**, and the transfer of cleaning liquid from filler pipe **12**, to piping **13**. Setting the pipe wall thickness for heat-storage piping **16**, **17** described above to, for example, 5 mm, permits a large volume of heat storage by increasing the heat capacity of heat-storage piping. (The drawing indicates pipe walls as cross sections, and thick walls are established slightly before distribution point **27**.) The number **32** located low in the sterilization chamber designates hot-air nozzles for vertically sealing the two lengthwise edges of tube-shaped web **4** by heating and fusing. The number **33** designates an air heater to send heated air to hot-air nozzles **32**.

The following describes sterilization of the packing machine filling section by hydrogen peroxide **5** liquid disinfectant. The drawing shows the condition for sterilizing the prescribed parts by creating a mixture **6** of hydrogen peroxide steam and heated air by spray-charging hydrogen peroxide into heat-storage piping **16**, **17**. The hydrogen peroxide **5** (ordinarily obtained as an aqueous solution) is a mixture of hydrogen peroxide ($H_2O_2$) and water with a minimum of 35% hydrogen peroxide ($H_2O_2$), and the most appropriate is a concentration of 35%. Prior to sterilization with this hydrogen peroxide **5**, heat-storage piping **16**, **17** is preheated with high-temperature heated air. In other words, air is compressed with air compressor **8** and its water separated with water separator **11** to yield dehydrated air **3** which enters heat-exchanger flow-path switching valve **29**, then enters from above said valve into high-temperature air heater **10** via side pipe **19a**, and is heated by the heated and engaged heater **10a**. The high-temperature (approximately 360°C) heated air travels through the heat exchanger without exchange of heat to flow distribution point **27**. From distribution point **27** during preheating prior to spray-charging hydrogen peroxide **5**, in relation to the side of heat-storage pipe **16** of filler pipe **12** and to the other side of sterile-air inlet pipe **14** and sterile chamber **30**, 2/3 of the flow volume of the high-temperature heated air described above is sent to the former side of heat-storage pipe **16** and 1/3 of the flow volume of said heated air is sent to the latter side of heat-storage pipe **17**. This enables efficient preheating of the prescribed parts for hydrogen peroxide sterilization. High-temperature heated air that passes distribution point **27** and flows into heat-storage pipe **16** on the filler-pipe side and into heat-storage pipe **17** on the other side heats the pipe walls for a prescribed period (15 to 20 seconds). The air that flowed into heat-storage pipe **16** on the filler-pipe side passes sterile-air valve **23b** of sterile production material valve device **23**, and is exhausted from valve **24** on pipe **34** which is only open during preheating. At this time, sterile-air valve **23b** of sterile production material valve device **23** has moved (upward from the condition in the drawing) and closed so that high-temperature heated air does not enter piping **13** connected to filler pipe **12**. Steam barrier valve **23c** and production material valve **23a** remain in the condition shown in the drawing, so flow of sterile production material **2** to the filler-pipe side is stopped, and sterile steam fills the steam barrier valve **23c** interior. The high-temperature heated air that flowed into heat-storage pipe **17** to heat its pipe walls is exhausted externally by moving heated-air flow-path transfer valve **26a** to the left (shown with imaginary lines) because the piston had moved to the right during preheating. In this way, because high-temperature heated air does not flow to filler pipe **12** and connecting piping **13**, sterile-air feed pipe **14** and connecting piping **15**, **15a**, and piping **15b** connected to the upper part of sterilization chamber **30**, temperature increase in parts requiring sterilization is completely prevented so that internal machine temperature (maximum 70° to 90°C) is maintained for operations prior to preheating described above such as preheating vertically sealing hot-air nozzles **32**.

After preheating for the prescribed period and after a sufficiently large heat storage takes place within heat-storage piping **16**, **17**, high-temperature heated air is sent continuously to said heat-storage piping **16**, **17**. Then, in a pressurized condition, hydrogen peroxide (aqueous solution) **5** which is a mixture of at least 35% hydrogen peroxide ($H_2O_2$) and water is sent from an unillustrated hydrogen peroxide tank by pump via piping **22** to divided nozzles **21**, and spray-charged into high-temperature heated air passing through venturi tubes **20** which are established on heat-storage piping **16**, **17**. A spraying time of 15 to 20 seconds is suitable, with an upper limit of 40 to 50 seconds permissible. Because nozzle openings are located at the throats **20a** of venturi tubes **20**, the high-temperature heated air passing said venturi tubes **20** increases its flow speed at said throats **20a** and pressure decreases while heat is restored from wall surfaces of heat-storage piping **16**, **17** to the hydrogen peroxide sprayed as a mist, causing instant vaporization. During spraying, heated air temperature changes from 320°C (spray started) to 200°C (spray ended). In addition, by establishing radial fins in heat-storage piping **16**, **17** described above, vaporization is made efficient without worry of recondensation because heat transfer from heat-storage piping **16**, **17** is facilitated. The vaporized hydrogen peroxide steam mixes with the high-temperature air flow and becomes a mixture **6** of hydrogen peroxide and air. Due to the absorption of the heat of vaporization during vaporization the temperature of mixture **6** attempts to greatly decrease, but instead is restored with a large volume of heat as it passes through heat-storage piping **16**, **17**, attaining a temperature relatively higher than when the dew point of mixture **6** is not restored with heat, whereby said mixture **6** traveling along heat-storage pipe **16** is conducted to filler pipe **12** via piping **13** to be sterilized and via sterile production material valve device **23**, and whereby said mixture **6** traveling along heat-storage pipe **17** is conducted through high-temperature air flow-path transfer valve **26** (in this case valve plug **26a** of said valve **26** is to the left as in the drawing) and from piping **15** in part to sterile-air inlet pipe **14** in tube-shaped web **4** via piping **15a** and in part to within sterilization chamber **30** from the upper part of said chamber **30** via piping **15b**.

At this time, because surface temperatures of piping to be sterilized **13**, **15**, **15a**, **15b**, filler pipe **12**, inner surfaces of sterile-air inlet pipe **14**, outer surface of sterile-air inlet pipe **14** inside sterilization chamber **30**, and outer surfaces of filler pipe **12** remain within the maximum range of 70° to 90°C around the heat source for the vertically sealing hot-air nozzles **32**, special cooling operations (ordinarily 10 to 15 minutes) are not required, and the hydrogen peroxide steam in the mixture described above clads and condenses as a thin layer on these surfaces to be sterilized which are below the dew point of the mixture described above. Valve **24** on pipe **34** connected to sterile-air valve **23b** of the sterile production material valve device is closed during the above operation. After the appropriate time passes, heated air of a prescribed temperature lower than the high-temperature heated air described above (to avoid the ill effects of high temperature on packing of the filler pipe, etc.) is used to evaporate the clad film of hydrogen peroxide disinfectant and complete sterilization. Hence, in this case, piston **29a** of heat-exchanging flow-path transfer valve **29** is up (shown with imaginary lines), and dehydrated air **3** emitted from water separator **11** enters said flow-path transfer valve **29** and travels through heat exchanger **19** from the lower part of said valve **29** to high-temperature air heater **10**. The high-temperature heated air passes through heat exchanger **19** again, but is cooled via heat-exchanging plates **19b** by dehydrated air **3** flowing into heat exchanger **19**, and, as heated air at a lower temperature than the high-temperature heated air for hydrogen peroxide spray-charging, said air is sent to piping and filler piping to be sterilized from piping distribution point **27** as described previously for sterilizing and drying hydrogen peroxide **5** condensed and clad to the surfaces to be sterilized, and evaporated hydrogen peroxide is exhausted along with heated air from the packing machine. During this time, the heated air temperature changes from an initial 200°C to 130°C after vaporization and drying. In this case, surfaces to be sterilized are not cooled so the initial temperature is higher for excellent drying, and residual amounts of hydrogen peroxide can be held to a minimum at production time.

## EFFECT OF THE PRESENT INVENTION

As the explanation above makes clear, the present invention furnishes the following effects.

In the manufacture of single-life packaging containers for the sterile-packing of liquid beverages such as milk, prior to the introduction of sterile liquid contents from the main product line pipe via a regulating valve device for its flow path, recently improved sterilizing systems have incorporated hydrogen peroxide steam for the sterilization of filler piping of contents and other parts to be sterilized, but these required the initial cooling of surfaces to be sterilized. Hence, time loss and a resulting increase in costs were unavoidable. By adopting the method of the present invention, highly concentrated conditions permit the generation of disinfectant steam such as hydrogen peroxide to provide superior condensation on the surfaces to be sterilized, and all cooling operations for the surfaces to be sterilized can be eliminated for increased operating efficiency and decreased costs. And because cooling operations on the surfaces to be sterilized are not carried out, good drying takes place which minimizes residual amounts of disinfectant for safer sterilization operations.

Establishing a maximum surface temperature range of 70° to 90°C is a suitable temperature for eliminating cooling operations, and by establishing a dew point of 90°C or greater for the mixture of air and liquid disinfectant, sterilization operations without cooling the surfaces to be sterilized and thus with less work can be carried out smoothly.

In addition, rapid vaporization occurs by opening nozzle openings at the throats of the venturi tubes in the heat-storage piping, and further, by making pipe walls thick in the heat-storage piping a large amount of heat storage is facilitated and this is effective for raising the dew point of the mixture of air and disinfectant. Establishing heat-exchanging parts such as radial fins efficiently raises the dew point of the mixture described above.

Moreover, by setting the spray-charging time for liquid disinfectant to 15 to 20 seconds, efficient sterilization operations can be carried out without waste at the packing machine. As liquid disinfectant, hydrogen peroxide is most suitable.

In execution of the method described above, by externally exhausting the heated air used for preheating heat-storage piping at the link between the heat-storage piping and piping to be sterilized, the temperature rise of surfaces to be sterilized is completely prevented, for efficient condensation of liquid disinfectant on the surfaces to be sterilized (disinfected).

**Claims**

1. A sterilization method for sterilizing surfaces of components of a machine for filling, dispensing and/or packing of liquids by using a liquid disinfectant, characterized by the steps of:
   – preheating a heat-storage piping (16,17) of a suitable heat capacity by transferring to it the heat of a super-heated airstream passing therethrough;
   – spraying a suitable amount of liquid disinfectant (5) into said super-heated airstream over a predetermined period of time and thereby vapourizing said liquid disinfectant and mixing said vapourized liquid disinfectant with said super-heated air and preventing a loss in temperature of said mixture (6) by transferring the heat stored in said heat-storage piping thereto:
   – conducting said mixture (6) of super-heated air and vapourized liquid disinfectant through said heat-storage piping and into contact with said components to be sterilized, which are connected to said heat-storage piping and which have a temperature on a level below the dew point temperature of said mixture thereby condensing a film of said liquid disinfectant onto the surfaces of said components;
   – stopping the supply of said mixture (6) of superheated air and vaporized liquid disinfectant;
   – conducting a further heated airstream through said heat-storage piping and into contact with said condensed film on said surfaces of said components thereby evaporating said film of liquid disinfectant.

2. A sterilization method as claimed in claim 1, wherein the surface maximum temperature at the surfaces of said components to be sterilized is on a level within a range from 70 to 90°C which is lower than the dew point temperature of said mixture of disinfectant and superheated air.

3. A sterilization method as claimed in claim 2, wherein the dew point temperature of said mixture of disinfectant and superheated air is higher than 90°C.

4. A sterilization method as claimed in any of claims 1 to 3, wherein spraying of liquid disinfectant into the heat-storage piping is carried out through the opening of a nozzle of a disinfectant supply pipe (22) directed to a throat portion of a venturi (21) tube located in the heat-storage piping.

5. A sterilization method as claimed in any of claims 1 to 4, wherein the wall thickness of the heat-storage piping is provided with a specific wall-thickness thicker than that of said components connected to said heat-storage piping.

6. A sterilization method as claimed in claim 5, wherein the heat-storage piping is provided with heat exchanging members like a radial fin.

7. A sterilization method as claimed in any of claims 1 to 6, wherein said spraying of liquid disinfectant is carried out over a period of 15 to 20 seconds.

8. A sterilization method as claimed in any of claims 1 to 7, wherein said liquid disinfectant is hydrogen peroxide.

9. A sterilization method as claimed in any of claims 1 to 8, wherein said superheated air is exhausted at a connecting portion (24) of said heat-storage piping and said components to be sterilized after preheating of said heat-storage piping.

**Patentansprüche**

1. Sterilisierungsverfahren zum Sterilisieren von Bauteile-Oberflächen einer Maschine zum Füllen, Abgeben und/oder Verpacken von Flüssigkeiten mittels eines flüssigen Desinfektionsmittels, **gekennzeichnet** durch die Schritte:
   – Vorheizen einer wärmespeichernden Rohrleitung (16,17) mit einer geeigneten Wärmekapazität durch Übertragen der Wärme eines durchströmenden, überhitzten Luftstroms;
   – Sprühen einer geeigneten Menge flüssigen Desinfektionsmittels (5) in den überhitzten Luftstrom, während eines vorbestimmten Zeitintervalls und dadurch Verdampfen des flüssigen Desinfektionsmittels, und Mischen des verdampften flüssigen Desinfektionsmittels mit der überhitzten Luft, und Verhindern eines Temperaturabfalls des Gemisches (6) durch Übertragen der in der wärmespeichernden Rohrleitung gespeicherten Wärme;
   – Führen des Gemisches (6) aus überhitzter Luft und verdampftem, flüssigem Desinfektionsmittel durch die wärmespeichernde Rohrleitung und in Kontakt mit den zu sterilisierenden Bauteilen, die mit der wärmespeichernden Rohrleitung verbunden sind und eine Temperatur auf einem Niveau unterhalb der Taupunkttemperatur des Gemisches aufweisen, um dadurch einen Film des flüssigen Desinfektionsmittels auf den Bauteile-Oberflächen niederschlagen;

– Beenden des Zuführens des Gemisches (6) aus überhitzter Luft und verdampftem, flüssigem Desinfektionsmittel;

– Führen eines weiteren erhitzten Luftstroms durch die wärmespeichernde Rohrleitung und in Verbindung mit dem niedergeschlagenen Film auf den Bauteile-Oberflächen, um dadurch den Film aus flüssigem Desinfektionsmittel verdampfen zu lassen.

2. Sterilisierungsverfahren nach Anspruch 1, dadurch **gekennzeichnet,** daß die maximale Oberflächentemperatur an den zu sterilierenden Bauteile-Oberflächen auf einem Niveau unterhalb des Bereiches von 70° bis 90° C ist, welches niedriger als die Taupunkttemperatur des Gemisches aus Desinfektionsmittel und überhitzter Luft ist.

3. Sterilisierungsverfahren nach Anspruch 2, dadurch **gekennzeichnet,** daß die Taupunkttemperatur des Gemisches aus Desinfektionsmittel und überhitzter Luft höher als 90° C ist.

4. Sterilisierungsverfahren nach den Ansprüchen 1 bis 3, dadurch **gekennzeichnet,** daß das Sprühen des flüssigen Desinfektionsmittels in die wärmespeichernde Rohrleitung durch die Öffnung einer Düse einer Desinfektionsmittel-Versorgungs-Leitung (22) durchgeführt wird, welche zu einem Versorgungsbereich eines Venturirohres (21) gerichtet ist, das in der wärmespeichernden Rohrleitung angeordnet ist.

5. Sterilisierungsverfahren nach den Ansprüchen 1 bis 4, dadurch **gekennzeichnet,** daß die Wanddicke der wärmespeichernden Rohrleitung eine spezielle Wanddicke ist, die dicker als die der mit der wärmespeichernden Rohrleitung verbundenen Bauteile ist.

6. Sterilisierungsverfahren nach Anspruch 5, dadurch **gekennzeichnet,** daß die wärmespeichernde Rohrleitung mit Wärmetauschelementen wie z.B. einer radialen Rippe versehen ist.

7. Sterilisierungsverfahren nach den Ansprüchen 1 bis 6, dadurch **gekennzeichnet,** daß das Sprühen des flüssigen Desinfektionsmittels über einen Zeitraum von 15 bis 20 Sekunden durchgeführt wird.

8. Sterilisierungsverfahren nach den Ansprüchen 1 bis 7, dadurch **gekennzeichnet** , daß das flüssige Desinfektionsmittel Wasserstoffperoxid ist.

9. Sterilisierungsverfahren nach den Ansprüchen 1 bis 8, dadurch **gekennzeichnet** , daß die überhitzte Luft an einem Verbindungsbereich (24) der wärmespeichernden Rohrleitung und der zu sterilisierenden Bauteile nach dem Vorheizen der wärmespeichernden Rohrleitung entweicht.

## Revendications

1. Procédé de stérilisation, pour la stérilisation de surfaces de composants d'une machine de remplissage avec des liquides, de distribution de liquides et/ou de conditionnement de liquides, au moyen d'un désinfectant liquide, caractérisé en ce qu'il comprend les étapes consistant :

– à préchauffer une canalisation d'accumu lation de chaleur (16, 17) ayant une capacité calorifique convenable par transfert à cette canalisation de la chaleur d'un courant d'air surchauffé passant à travers cette canalisation ;

– à pulvériser une quantité convenable de désinfectant liquide (5) dans ledit courant d'air surchauffé pendant un temps prédéterminé et vaporiser ainsi ledit désinfectant liquide et mélanger ledit désinfectant liquide vaporisé à l'air surchauffé et empêcher un abaissement de température dudit mélange (6) par transfert de la chaleur stockée dans ladite canalisation d'accumulation de chaleur à ce mélange ;

– à conduire ledit mélange (6) d'air surchauffé et de désinfectant liquide vaporisé à travers ladite canalisation d'accumulation de chaleur et en contact avec lesdits composants à stériliser, qui sont connectés à ladite canalisation d'accumulation de chaleur et qui possèdent une température égale à une valeur inférieure au point de rosée dudit mélange, ce qui provoque la condensation d'un film dudit désinfectant liquide sur les surfaces desdits composants;

– à interrompre l'introduction dudit mélange (6) d'air surchauffé et de désinfectant liquide vaporisé ;

– à conduire un nouveau courant d'air chauffé à travers ladite canalisation d'accumulation de chaleur et en contact avec ledit film condensé sur lesdites surfaces desdits composants, ce qui provoque l'évaporation dudit film de désinfectant liquide.

2. Procédé de stérilisation suivant la revendication 1, dans lequel la température maximale de surface, au niveau des surfaces des composants à stériliser, est égale à une valeur comprise dans l'intervalle de 70 à 90°C, qui est inférieure au point de rosée du mélange de désinfectant et d'air surchauffé.

3. Procédé de stérilisation suivant la revendication 2, dans lequel le point de rosée du mélange de désinfectant et d'air surchauffé est supérieur à 90°C.

4. Procédé de stérilisation suivant l'une quelconque des revendications 1 à 3, dans lequel la pulvérisation de désinfectant liquide dans la canalisation d'accumulation de chaleur est effectuée par l'orifice d'une buse d'un conduit d'alimentation en désinfectant (22) dirigé vers un étranglement d'un tube venturi (21) situé dans la canalisation d'accumulation de chaleur.

5. Procédé de stérilisation suivant l'une quelconque des revendications 1 à 4, dans lequel l'épaisseur de paroi de la canalisation d'accumulation de chaleur possède une valeur spécifique supérieure à celle de l'épaisseur de paroi des composants connectés à ladite canalisation d'accumulation de chaleur.

6. Procédé de stérilisation suivant la revendication 5, dans lequel la canalisation d'accumulation de chaleur est munie d'éléments d'échange de chaleur analogues à une ailette radiale.

7. Procédé de stérilisation suivant l'une quelconque des revendications 1 à 6, dans lequel la pulvérisation de désinfectant liquide est effectuée pendant un temps de 15 à 20 secondes.

8. Procédé de stérilisation suivant l'une quelconque des revendications 1 à 7, dans lequel le désinfectant liquide est le peroxyde d'hydrogène.

9. Procédé de stérilisation suivant l'une quelconque des revendications 1 à 8, dans lequel l'air surchauffé est évacué au niveau d'une portion de connexion (24) de la canalisation d'accumulation de chaleur et des composants à stériliser après préchauffage de ladite canalisation d'accumulation de chaleur.